# EUROPÄISCHE PATENTSCHRIFT

(11) **EP 2 129 650 B1**
(45) Veröffentlichungstag und Bekanntmachung des Hinweises auf die Patenterteilung: **24.08.2016**
(21) Anmeldenummer: 08717908.1
(22) Anmeldetag: 17.03.2008
(51) Int. Cl.: C07C 51/50, C07C 51/47, C07C 51/42, C07C 57/04, C07C 45/78, C07C 45/79, C07C 45/86, C07C 47/22, C07C 67/48, C07C 67/56, C07C 67/62, C07C 69/54

(54) **VERFAHREN ZUR LAGERUNG EINER UNTER DEN BEDINGUNGEN DER LAGERUNG FLÜSSIGEN MONOMERENPHASE**
METHOD FOR STORING A MONOMER PHASE THAT IS LIQUID UNDER STORAGE CONDITIONS
PROCEDE DE STOCKAGE D'UNE PHASE MONOMERE LIQUIDE DANS LES CONDITIONS DE STOCKAGE

(30) Priorität: 23.03.2007 DE 102007014606; 23.03.2007 US 896559 P
(43) Veröffentlichungstag der Anmeldung: 09.12.2009
(73) Patentinhaber: BASF SE, 67056 Ludwigshafen am Rhein (DE)
(72) Erfinder: HEILEK, Jörg, 69245 Bammental (DE); HAMMON, Ulrich, 68163 Mannheim (DE); SCHLIEPHAKE, Volker, 67105 Schifferstadt (DE); MÜLLER-ENGEL, Klaus Joachim, 76297 Stutensee (DE); BLUM, Till, 67346 Speyer (DE)
(74) Vertreter: BASF IP Association
(86) Internationale Anmeldenummer: PCT/EP2008/053173
(87) Internationale Veröffentlichungsnummer: WO 2008/116776

(56) Entgegenhaltungen:
- EP-A- 1 361 203
- DE-A1- 10 219 089
- US-A1- 2002 008 064

## Beschreibung

Vorliegende Erfindung betrifft ein Verfahren zur Lagerung einer unter den Bedingungen der Lagerung flüssigen Monomerenphase, deren Gehalt an dem Monomeren ≥ 95 Gew.-% beträgt, in einem Lagerbehälter, wobei das Monomere ein Monomeres aus der Gruppe bestehend aus Acrolein, Methacrolein, Acrylsäure, Methacrylsäure, Ester aus Acrylsäure und einem 1 bis 12 C-Atome aufweisenden Alkohol sowie Ester aus Methacrylsäure und einem 1 bis 12 C-Atome aufweisenden Alkohol ist und die flüssige Monomerenphase durch Kondensation aus einer gasförmigen Phase heraus oder durch Aufschmelzen einer kristallinen Phase erzeugt wurde, dadurch gekennzeichnet, dass die flüssige Monomerenphase auf dem Weg von ihrer Erzeugung in den Lagerbehälter wenigstens einer Trennoperation zur Abtrennung wenigstens einer Teilmenge von in der flüssigen Monomerenphase gelöst enthaltenem Polymerisat des Monomeren unterworfen wird und der Lagerbehälter eine Vorrichtung aufweist, mit Hilfe derer eine Teilmenge der gelagerten flüssigen Monomerenphase entnommen, über einen Wärmeaustauscher geführt und anschließend in den Lagertank rückgeführt werden kann.

Ferner betrifft vorliegende Erfindung ein Verfahren zur Entnahme von flüssiger Monomerenphase aus dem Lagerbehälter.

Gemäß den vorstehenden Ausführungen beinhaltet der Begriff Monomer(e) in dieser Schrift Acrolein, Methacrolein, Acrylsäure, Methacrylsäure, Ester aus Acrylsäure und einem 1 bis 12 C-Atome aufweisenden Alkohol sowie Ester aus Methacrylsäure und einem 1 bis 12 C-Atome aufweisenden Alkohol. Als Alkohole kommen dabei sowohl einwertige (weisen eine -OH-Gruppe auf) als auch mehrwertige (weisen mehr als eine -OH-Gruppe auf) Alkohole in Betracht. Zu diesen Alkoholen gehören insbesondere 1 bis 12, vorzugsweise 1 bis 8 C-Atome aufweisende ein- und mehrwertige Alkanole. Diese Definition beinhaltet nicht in notwendiger Weise, dass die Herstellung dieser Ester durch Reaktion der entsprechenden Alkohole mit der jeweiligen Säure erfolgen muss. Vielmehr kommen als Herstellverfahren auch andere Reaktionen wie z. B. Umesterungen oder Additionsreaktionen in Betracht.

Als beispielhafte Monomere seien genannt Methylacrylat, Ethylacrylat, n-Butylacrylat, iso-Butylacrylat, tert.-Butylacrylat, 2-Ethylhexylacrylat, Hydroxyethylacrylat, Hydroxypropylacrylat, Hydroxymethylmethacrylat, Hydroxypropylmethacrylat, Methylmethacrylat, Ethylmethacrylat, n-Butylmethacrylat 2-Propylheptylacrylat und tert.-Butylmethacrylat.

Üblicherweise werden Monomere durch chemische Synthese erzeugt.

Dabei werden sie jedoch nicht unmittelbar in reiner Form befindlich, sondern als Bestandteil von gasförmigen oder flüssigen Produktgemischen erhalten, aus denen sie abgetrennt werden müssen. Diese Abtrennung wird in der Regel unter Anwendung thermischer Trennverfahren wie z.B. Absorption, Desorption, fraktionierende Kondensation, Extraktion, Kristallisation, Adsorption usw., bzw. unter Anwendung von Kombinationen verschiedener solcher thermischer Trennverfahren vorgenommen (vgl. z.B. DE-A 19 924 533, WO 2004/035 514, EP-A 1 388 533, EP-A 778 225, EP-A 1 041 062, EP-A 982 287, EP-A 982 288, WO 01/96 271, DE-A 10 336 386, DE-A 19 631 645, DE-A 19 501 325, EP-A 982 289, DE-A 19 838 845, WO 02/076 917, EP-A 1 695 954, EP-A 695 736, EP-A 778 225, EP-A 1 041 062, US 2004/0 242 826, EP-A 792 867, EP-A 784 046, EP-A 695 736, EP-A 112 5912, EP-A 1 097 741, WO 00/45 928, DE-A 2 246 480, DE-A 2 362 373, US-A 5,637,222 und der in diesen Schriften zitierte Stand der Technik).

Als Reinprodukt wird in der Regel eine flüssige Monomerenphase erhalten, deren Gehalt an dem jeweiligen Monomeren ≥ 95 Gew.-% beträgt.

Üblicherweise besteht der letzte Schritt auf dem Weg vom Produktgemisch zum Reinprodukt aus einer Kondensation aus einer gasförmigen Phase heraus oder aus dem Aufschmelzen einer kristallinen Phase. Beispielsweise kann die Kondensation in einer Rektifikationskolonne oberhalb der Zufuhrstelle des (in der Rektifikationskolonne) aufzutrennenden Gemischs in die Rektifikationskolonne aus den in der Rektifikationskolonne aufsteigenden Dämpfen heraus erfolgen und das Reinprodukt oberhalb des vorgenannten Zulaufs aus der Rektifikationskolonne entnommen werden. Dabei kann das aufzutrennende Gemisch in die Rektifikationskolonne sowohl flüssig (Rektifikation) als auch gasförmig (fraktionierende Kondensation) zugeführt werden.

In beiden Fällen, d.h., sowohl dann, wenn die flüssige Monomerenphase durch Kondensation aus einer gasförmigen Phase heraus, als auch dann, wenn die flüssige Monomerenphase durch Aufschmelzen einer kristallinen Phase (von Kristallisat) erzeugt wird, fällt sie normalerweise so an, dass sie bei visueller Betrachtung (d. h., bei Betrachtung mit bloßem menschlichem Auge) frei von Feststoffen ist. Dabei enthält die flüssige Monomerenphase üblicherweise radikalische Polymerisationsinhibitoren (Inhibitoren zur Unterdrückung unerwünschter radikalischer Polymerisation) gelöst, die regelmäßig im Verlauf ihrer Erzeugung und/oder als Abschluss einer solchen Erzeugung zudosiert werden. Im Fall einer Erzeugung durch Aufschmelzen einer kristallinen Phase kann das Polymerisationsinhibitorsystem aber auch bereits in der kristallinen Phase enthalten sein.

Die wie beschrieben erzeugte flüssige Monomerenphase wird als gewünschtes Reinprodukt anschließend normalerweise einem Lagerbehälter zugeführt, in welchem sie flüssig gelagert verbleibt, bis von ihr aus selbigem zu Zwecken der Verwendung in chemischen Reaktionen bzw. zu Zwecken des Transports entnommen wird. Üblicherweise werden solche Behälter auch als Tank bzw. auch als Lagertank bezeichnet. Das von einer fluiden Phase einnehmbare Innenvolumen eines Lagertanks (der in typischer Weise ruht) beträgt in typischer Weise 100 m³ bis 10 000 m³, häufig 200 m³ bis 1000 m³ und charakteristisch 500 m³ (vgl. z.B. Research Disclosure Database Number 513001, published in January 2007). Zum Zweck der Einhaltung der erwünschten Lagertemperatur weist ein Lagertank in der Regel eine Vorrichtung auf, mit Hilfe derer (z. B. kontinuierlich) eine Teilmenge der gelagerten flüssigen Monomerenphase entnommen, über einen Wärmeaustauscher geführt und anschließend in den Lagertank rückgeführt wird bzw. rückgeführt werden kann.

Da die vorgenannten Polymerisationsinhibitorsysteme ihre volle Wirkung normalerweise nur im Beisein von molekularem Sauerstoff (der seinerseits selbst Inhibitor ist) entfalten, werden die flüssigen Monomerenphasen üblicherweise unter einer molekularen Sauerstoff enthaltenden Gasatmosphäre gelagert (vgl. z.B. WO 2005/049 543 sowie US-A 6,910,511). D.h., das von einer fluiden Phase einnehmbare Innenvolumen des Lagertanks wird von der im Lagertank gelagerten flüssigen Monomerenphase nur teilweise ausgefüllt und das dabei verbleibende restliche einnehmbare Innenvolumen des Lagerbehälters von einer molekularen Sauerstoff enthaltenden Gasphase eingenommen. Mit Hilfe von Mischvorrichtungen wird in der Regel dafür Sorge getragen, dass die flüssige Monomerenphase an dem in ihr gelösten molekularen Sauerstoff nicht verarmt.

Aus Gründen der Inhibierung einer unerwünschten radikalischen Polymerisation der im Lagertank gelagerten flüssigen Monomerenphase ist ein möglichst hoher Gehalt an molekularem Sauerstoff in der Gasphase des Lagerbehälters wünschenswert. Nachteilig an einem hohen Sauerstoffgehalt der vorgenannten Gasphase ist jedoch, dass sie in notwendiger Weise auch verdampftes Monomer enthält, und Mischungen aus Monomer und molekularem Sauerstoff explosiv sein können (vgl. WO 2004/007 405, DE-A 102 004 034 515, WO 2005/049 543, Research Disclosure Database Number 513 001, published in January 2007 sowie Research Disclosure Database Number 513 002, published in January 2007). Insgesamt wird daher eine Minimierung des Sauerstoffgehalts der Gasphase im Lagertank angestrebt. Besonders vorteilhaft liegt sie unter der sogenannten Sauerstoffgrenzkonzentration (vgl. WO 2004/007 405), unterhalb derer ein explosives Verhalten des Gasgemisches nicht möglich ist.

DE 102 19 089 offenbart ein Verfahren zum Transport und/oder zur Lagerung von Acrylsäure.

In gleicher Weise ist aber auch ein möglichst geringer Gehalt der gelagerten flüssigen Monomerenphase an radikalischem Polymerisationsinhibitor wünschenswert. Dies ist zum einen darin begründet, dass es sich bei Polymerisationsinhibitoren um vergleichsweise kostspielige Wirkstoffe handelt. Zum anderen vermögen die Monomeren selbst und/oder deren spätere Reaktionsprodukte häufig mit den Inhibitoren unter Ausbildung von farbintensiven Reaktionsprodukten zu reagieren, was in der Regel unerwünscht ist. Weiterhin wirken sich die Polymerisationsinhibitoren bei einer Verwendung der gelagerten flüssigen Monomerenphase in radikalischen Polymerisationsreaktionen normalerweise nachteilig aus (z. B. auf die Produktqualität).

Vor dem vorgenannten Hintergrund bestand die Aufgabe der vorliegenden Erfindung insbesondere darin, ein Verfahren zur Lagerung einer unter den Bedingungen der Lagerung flüssigen Monomerenphase, deren Gehalt an dem jeweiligen Monomeren ≥ 95 Gew.-% beträgt, in einem Lagerbehälter zur Verfügung zu stellen, das unter Anwendung einer verringerten Zugabe von eine radikalische Polymerisation der Monomeren inhibierenden Stoffen eine sichere Lagerung der flüssigen Monomerenphase ermöglicht.

Demgemäß wurde ein Verfahren zur Lagerung einer unter den Bedingungen der Lagerung flüssigen Monomerenphase, deren Gehalt an dem Monomeren ≥ 95 Gew.-% beträgt, in einem Lagerbehälter, wobei das Monomere ein Monomeres aus der Gruppe bestehend aus Acrolein, Methacrolein, Acrylsäure, Methacrylsäure, Ester aus Acrylsäure und einem 1 bis 12 C-Atome (vorzugsweise 1 bis 8 und besonders bevorzugt 1 bis 4 C-Atome) aufweisenden Alkohol sowie Ester aus Methacrylsäure und einem 1 bis 12 C-Atome (vorzugsweise 1 bis 8 und besonders bevorzugt 1 bis 4 C-Atome) aufweisenden Alkohol ist und die flüssige Monomerenphase durch Kondensation aus einer gasförmigen Phase heraus oder durch Aufschmelzen einer kristallinen Phase erzeugt wurde, gefunden, das dadurch gekennzeichnet ist, dass die flüssige Monomerenphase auf dem Weg von ihrer Erzeugung in den Lagerbehälter wenigstens einer Trennoperation zur Abtrennung wenigstens einer Teilmenge (vorzugsweise wenigstens 25 Gew.-%, besser wenigstens 50 Gew.-%, noch besser wenigstens 75 Gew.-% und am besten die Gesamtmenge (insbesondere der unten genannten relativen Molekulargewichte)) von in der flüssigen Monomerenphase gelöst enthaltenem Polymerisat des Monomeren unterworfen wird. Der Begriff Polymerisat soll hier auch Oligomerisat umfassen. In erster Linie ist darunter durch (unerwünschte) radikalische Polymerisation erzeugtes Polymerisat/Oligomerisat gemeint.

Der Wortlaut "in der flüssigen Monomerenphase gelöst enthaltenem Polymerisat" soll in dieser Schrift sowohl "molekular gelöst" als auch "kolloidal gelöst" umfassen. Beides aber nur insoweit, als die gelöste Form in der zu lagernden flüssigen Monomerenphase visuell (d.h., mit bloßem menschlichem Auge) nicht wahrnehmbar ist.

Eigene Untersuchungen haben ergeben, dass insbesondere derartiges gelöstes Polymerisat (im Unterschied zu makroskopisch sichtbarem Polymerisat) eine ausgeprägt polymerisationsfördernde Aktivität aufweist. Dies gilt vor allem bei auf atomaren Wasserstoff bezogenen relativen Molekulargewichten von ≥ 1000, bzw. ≥ 2000 bzw. ≥ 3000 des gelösten Polymerisats.

Als Trennoperation zur Abtrennung von in der zu lagernden flüssigen Monomerenphase gelöst enthaltenem Polymerisat kommen insbesondere alle mechanischen Trennoperationen in Betracht, die zur Abtrennung von feinstteiligen Feststoffen von Flüssigkeiten geeignet sind. Diesbezüglich erfindungsgemäß besonders geeignet sind filtrierende und/oder zentrifugierende Trennoperationen. Dazu zählen im besonderen die Mikrofiltration, die Ultrafiltration (Nanofiltration) und das Ultrazentrifugieren (die Massendichten von oligomerisiertem und/oder polymerisiertem Monomer sind von der Massendichte des entsprechenden Monomeren ebenso ausreichend verschieden wie die jeweiligen Volumenausdehnungen; bei 25 °C und 1 atm beträgt die Massendichte von monomerer Acrylsäure beispielsweise 1,05 g/cm³ und von polymerisierter Acrylsäure 1,54 g/cm³; der Unterschied in der Massendichte fußt insbesondere auf dem erhöhten Raumbedarf der Elektronenwolke einer ungesättigten Doppelbindung; die oligo- und/oder polymerisierten Monomeren reichern sich am Mantel der Ultrazentrifuge an und können von selbigem kontinuierlich abgeschält werden. Selbstverständlich können auch chromatographische sowie osmotische Verfahren als erfindungsgemäße Trennoperation angewendet werden.

Erfindungsgemäß bevorzugt wird man das in der zu lagernden flüssigen Monomerenphase (visuell nicht wahrnehmbar) gelöst enthaltene Polymerisat durch Filtration von der flüssigen Monomerenphase abtrennen. Als Filtermaterialien können dabei z.B. Filtervliese, Filtergewebe, Faserschichten, Sintermaterialien oder Schüttschichten (z.B. aus feinteiligen Quarzmaterialien, Kieselgur, Aktivkohle, Zeolithe) verwendet werden.

Grundsätzlich können für den erfindungsgemäßen Anwendungszweck z.B. Beutelfilter oder Kerzenfilter eingesetzt werden. Die erfindungsgemäße Filtrationsaufgabe kann dabei sowohl mit genähten als auch mit voll verschweißten, vorzugsweise mehrlagigen Filterbeuteln aus unterschiedlichen Materialien gelöst werden. Als solche Materialien kommen z.B. Edelstahl, Polypropyplen, Cellulose, Polyester, Metallgewebe (Edelstahl wie z.B. Chrom-Nickel-Edelstahl) sowie phenolharzgebundene Acrylfasern in Betracht. Erfindungsgemäß besonders bevorzugtes Material für erfindungsgemäß zu verwendende Filter (sowohl Filterbeutel als auch Filterkerzen) ist Polypropylen. Erfindungsgemäß verwendbare Filterkerzen können jedoch z.B. auch aus Aktivkohle gefertigt sein. Grundsätzlich kommen als erfindungsgemäß geeignete Filterkerzen sowohl Wickelkerzen, Meltblown-Kerzen und harzgebundene Filterkerzen in Betracht. Sowohl die Filterbeutel als auch die Filterkerzen können erfindungsgemäß sowohl in Einfach- und in Mehrfach-Filtergehäusen zur Anwendung kommen. Als Gehäusematerial kommt z.B. Polypropylen, Edelstahl und C-Stahl in Betracht. Erfindungsgemäß bevorzugt ist die Anwendung von Mehrfach-Gehäusen, in denen bis zu ca. 40 einzelne Filterbeutel eingesetzt werden können und die Durchflussmengen an flüssiger Monomerenphase von bis zu 1000 m³/h ermöglichen. Einbauhilfen oder Beutelniederhalter verbessern den korrekten Sitz der Filterbeutel. Beispielsweise verhindern sie sicher ein "Aufschwimmen" durch unkontrollierten Rückstau. Ebenso wird ein Platzen des Filterbeutels verhindert. Spezifische "Kragen" sorgen für perfekten und festen Sitz des Beutelniederhalters im Filterbeutel (kein Durchscheuern oder Erosion des Filterbeutels).

Erfindungsgemäß bevorzugt werden Filtermaterialien (Filtermedien bzw. Filtertypen) eingesetzt, deren Abscheidegrad (retention efficiency) für Partikel mit einem Teilchendurchmesser von ≥ 30 µm wenigstens 70 %, vorzugsweise wenigstens 80 %, besonders bevorzugt wenigstens 90 % bzw. wenigstens 95 % und mehr beträgt.

Besonders bevorzugt werden beim erfindungsgemäßen Verfahren Filtermaterialien (Filtermedien bzw. Filtertypen) eingesetzt, deren Abscheidegrad für Partikel mit einem Teilchendurchmesser von ≥ 20 µm wenigstens 70 %, vorzugsweise wenigstens 80 %, besonders bevorzugt wenigstens 90 % bzw. wenigstens 95 % und mehr beträgt.

Ganz besonders bevorzugt werden beim erfindungsgemäßen Verfahren Filtermaterialien (Filtermedien bzw. Filtertypen) eingesetzt, deren Abscheidegrad für Partikel mit einem Teilchendurchmesser von ≥ 10 µm wenigstens 70 %, vorzugsweise wenigstens 80 %, besonders bevorzugt wenigstens 90 % bzw. wenigstens 95 % und mehr beträgt.

Noch besser werden beim erfindungsgemäßen Verfahren Filtermaterialien (Filtermedien bzw. Filtertypen) eingesetzt, deren Abscheidegrad für Partikel mit einem Teilchendurchmesser von ≥ 5 µm wenigstens 70 %, vorzugsweise wenigstens 80 %, besonders bevorzugt wenigstens 90 % bzw. wenigstens 95 % und mehr beträgt.

Am besten werden beim erfindungsgemäßen Verfahren Filtermaterialien (Filtermedien bzw. Filtertypen) eingesetzt, deren Abscheidegrad für Partikel mit einem Teilchendurchmesser von ≥ 1 µm wenigstens 70 %, vorzugsweise wenigstens 80 %, besonders bevorzugt wenigstens 90 % bzw. wenigstens 95 % und mehr beträgt.

In der Regel werden für das erfindungsgemäße Verfahren (sowie für das später in dieser Schrift noch ausgeführte Entnahmeverfahren) jedoch Filtermaterialien (Filtermedien bzw. Filtertypen) eingesetzt, deren Abscheidegrad für Partikel mit einem Teilchendurchmesser von 0,5 µm bei ≤ 90 %, vorzugsweise bei ≤ 80 % liegt. Dadurch werden für das erfindungsgemäße Verfahren in der Regel befriedigende Raum-Zeit-Ausbeuten gewährleistet.

Die vorgenannten Prozentsätze beziehen sich jeweils auf die Gesamtpartikelanzahl der jeweiligen Partikelgröße. Ferner beziehen sich vorgenannte Prozentsätze auf eine Flächenbelastung von 20 l/(m²·min), Wasser als Trägermedium für die Partikel, eine Temperatur von 20°C und eine Druckdifferenz < 50 mbar, sowie Teststaub gemäß ISO 12103-1 A3 als Partikelart.

Die Testausführung erfolgt in Anlehnung an den French standard NF 45-303 (vgl. Filtration & Separation (Filtr. sep.) ISSN 0015-1882 CODEN FSEPAA Filtration and Separation, 1997, vol. 34, n°3, pp. 217-223).

Alle vorgenannten Eigenschaften erfüllen beispielsweise ACCUGAF™ Filterbeutel der Firma Eaton Filtration, LLC., 900 Fairmount Avenue, Elizabeth, New Jersey 07207 (USA) der Filtermodelle AGF-51, AGF-53, AGF-55, AGF-57 und AGF-59. Sie sind aus schmelzgeblasenem Polypropylen in verschweißter Form gefertigt. Sie weisen keine Bindemittel, keine grenzflächenaktiven Substanzen und keine Additive (z.B. klebende Harze) auf. Derartige, nur aus Polypropylen gefertigte, Filtermaterialen (Filtermedien , Filtertypen) erweisen sich für das erfindungsgemäße Verfahren als besonders geeignet, da sie die erfindungsgemäß zu lagernden flüssigen Monomerphasen in besonders geringfügiger Weise zu unerwünschter radikalischer Polymerisation reizen.

Die höchsten Abscheidegrade werden dabei in Anwendung des Typs AGF-51 erreicht (wenigstens 90 % für Partikeldurchmesser ≥ 1 µm). Mit zunehmender numerischer Kennzahl nehmen die Abscheidegrade ab. Für den Typ AGF-59 liegt der Abscheidegrad für Parikeldurchmesser ≥ 30 µm jedoch noch immer oberhalb von 90 %.

Alternativ können für das erfindungsgemäße Verfahren auch Filterbeutel PROGAF™ oder LOFCLEAR™ der Firma Eaton verwendet werden. Bei den LOFCLEAR Filterbeuteln handelt es sich um Filterbeutel aus mehrlagigem Filtermaterial und bei den PRO-GAF™ Filterbeuteln handelt es sich um Filterbeutel aus besonders hoch effizientem Filtermedium, mit dem effektive Partikelreduktionen bis in den Submikron-Bereich erreicht werden können. LOFCLEAR Filterbeutel sind ebenfalls aus 100 % Polypropylen gefertigt.

Alternativ können für ein erfindungsgemäßes Verfahren die Hochleistungs-Beutelfilter der Baureihe "HP" (insbesondere die Beutelfilter HPC, HPB und HPA) der Pall GmbH, Philipp-Reis-Straße 6, D-63303 Dreieich verwendet werden. Sie bestehen aus drei übereinander angeordneten Polypropylen-Vliesen; einem äußeren Stützvlies, das eine gute mechanische Festigkeit des Beutelfilters gewährleistet, dem mittleren aktiven Filtervlies und einem inneren Schutzvlies, das als Vorfilter fungiert.

Günstig sind Beutelfilter, deren Differenzdruck im Neuzustand bei einer Belastung mit 10 (vorzugsweise mit 20) m³/h • m² 20°C aufweisendem Wasser ≤ 100 mbar beträgt. In der Regel liegt der vorgenannte Wert jedoch bei ≥ 5 mbar.

Als erfindungsgemäß geeignet seien weiterhin aus Polypropylen gefertigte Kerzenfilter der Firma FUHR GmbH (D-55270 Klein-Winternheim bei Mainz, Am Weinkastell 14) erwähnt. Dazu zählen insbesondere die acuraProgard Membranfilterkerzen, die ebenfalls vollständig aus Polypropylen gefertigt sind. Sie weisen eine thermische Schweißkonstruktion auf und sind dadurch frei von Kleber und Bindemitteln. Da ihre Filtermatrix eine zweilagige Struktur aufweist, können bei ihrer Verwendung äußerst lange Standzeiten erreicht werden.

acuraPrograd Kerzenfilter sind mit Abscheideraten von wenigstens 99,9 % für Partikeldurchmesser ≥ 0,2 µm, bzw. ≥ 0,45 µm, bzw. ≥ 1 µm, bzw. ≥ 5 µm, bzw. ≥ 10 µm oder ≥ 20 µm erhältlich.

Die Temperatur der erfindungsgemäß zu filtrierenden Monomerenphase sollte insbesondere bei Verwendung von Filtermedien aus Polypropylen ≤ 95°C betragen.

Vorzugsweise liegt vorgenannte Temperatur bei ≤ 80°C, besonders bevorzugt bei ≤ 60°C, ganz besonders bevorzugt bei ≤ 40°C und am besten bei ≤ 30 °C bzw. ≤ 25°C. In günstigen Fällen kann sie bis zu 0 °C betragen.

Im übrigen wird die wenigstens eine Trennoperation des erfindungsgemäßen Verfahrens generell vorzugsweise bei Temperaturen ≤ 95°C, bevorzugt ≤ 80°C, vorteilhaft ≤ 60°C, besonders vorteilhaft ≤ 40°C und am besten bei ≤ 30 °C bzw. ≤ 25°C durchgeführt. In günstigen Fällen kann sie bis zu 0 °C betragen.

Bei einer wie beschrieben erfindungsgemäß durchzuführenden Filtration können Differenzdrucke von ≥ 10 mbar bis 5 bar angewendet werden.

Vorzugsweise liegen vorgenannte Differenzdrucke bei ≥ 20 mbar und ≤ 3 bar, besonders bevorzugt bei ≥ 20 mbar und ≤ 2 bar, bzw. ≤ 1,5 bar. Werden höhere Differenzdrucke erforderlich, sollten die Filter ausgetauscht werden. Selbstverständlich kommen für eine erfindungsgemäße Filtration aber auch Filterkerzen aus Polytetrafluorethylen (z. B. die acuraVent AVF Filterkerzen der FUHR GmbH), aus Borasilikatfasern (z. B. die acuraVent AFG-GG Filterkerzen der FUHR GmbH), aus Polyethersulfon (z. B. die acuraFine AFS Filterkerzen der FUHR GmbH), sowie aus Nylon (z. B. die acuraPrograd Filterkerzen der FUHR GmbH) in Betracht.

Selbstverständlich können für eine erfindungsgemäße Filtration auch die acuraBag Filterbeutel der FUHR GmbH, insbesondere diejenigen, die aus Polypropylen gefertigt sind, verwendet werden. Erfindungsgemäß geeignet sind aber auch die ACCUFIT^{®} und die ULTRAFIT^{®}-Filterbeutel der FUHR GmbH. Sie sind erfindungsgemäß vorteilhaft ebenfalls aus Polypropylen in verschweißter Ausführung gefertigt.

In der Regel sind erfindungsgemäß geeignete Filterbeutel aus mehreren Filterlagen gefertigt.

Erfindungsgemäß vorteilhaft ist, wenn sich das für ein erfindungsgemäß eingesetztes Verfahren eingesetzte Filtermedium unter der Belastung mit zu filtrierender flüssiger Monomerenphase nicht wesentlich ausdehnt. Vorgenannte Belastung wird in der Regel 2 bis 20 m³/(h • m²) betragen.

In der Regel wird man die zu filtrierende flüssige Monomerenphase bei Verwendung von Filterbeuteln oder Filterkerzen von innen nach außen durch den Filtertyp führen. Grundsätzlich kann bei Verwendung von Filterkerzen die Führung der flüssigen Monomerenphase auch von außen nach innen erfolgen.

Typische Porengrößen der filteraktiven Schicht von erfindungsgemäß zu verwendenden Filtermaterialien betragen 0,1 bis 300 µm, vorzugsweise 1 bis 200 µm bzw. 1 bis 100 µm und besonders bevorzugt 1 bis 50 µm bzw. 1 bis 5 µm. Wie bereits erwähnt, werden erfindungsgemäß vorteilhaft mehrere Lagen (z. B. 3, oder 5, oder 7 Lagen) übereinander angewendet.

Die Filtration kann grundsätzlich als Druck- oder als Vakuumfiltration praktiziert werden. Vorzugsweise wird sie als Druckfiltration durchgeführt. Selbstverständlich kann sie auch zentrifugierend praktiziert werden.

Das erfindungsgemäße Verfahren eignet sich insbesondere dann, wenn der Gehalt der flüssigen Monomerenphase an dem Monomeren ≥ 96 Gew.-%, oder ≥ 97 Gew.-%, oder ≥ 98 Gew.-%, oder ≥ 99 Gew.-%, oder ≥ 99,5 Gew.-%, oder ≥ 99,7 Gew.-%, oder ≥ 99,9 Gew.-% beträgt. Das Vorgenannte und alle Aussagen in dieser Schrift sind insbesondere dann zutreffend, wenn das Monomere Acrylsäure ist (insbesondere wenn die flüssige Monomerenphase eine Reinacrylsäure ist).

Ist ein erfindungsgemäß verwendeter Filtertyp erschöpft, kann er durch einen frischen Filter ersetzt werden. Selbstredend kann der Filter zielgerichtet als Wechselfilter gestaltet werden. Durch Waschen mittels wässrigem Alkalihydroxid (z. B. Kaliumhydroxid und/oder Natriumhydroxid) und nachfolgendes Neutralwaschen mit reinem Wasser können erfindungsgemäß erschöpfte Filter regeneriert werden. Insbesondere aus Polypropylen gefertigte Filter können nach ihrer Erschöpfung problemlos verbrannt werden.

Als eine unerwünschte radikalische Polymerisation des in der flüssigen Monomerenphase befindlichen Monomeren (z. B. ausgelöst durch Temperatur, Licht oder sonstige spontan ausgelöste Radikalbildung) inhibierende Wirkstoffe kann die erfindungsgemäß zu lagernde flüssige Monomerenphase jeden der im Stand der Technik für diesen Zweck bekannten Polymerisationsinhibitoren gelöst enthalten. Bevorzugt verwendete Polymerisationsinhibitoren sind p-Methoxyphenol (MEHQ), Phenothiazin, Hydrochinone, Phenol (z. B. 2,4-Dimethyl-6,6-butylphenol), Chinone, Butylbrenzkatechin, Diphenylamin, p-Phenylendiamine, Nitroxyl-Radikale (z. B. 4-Hydroxy-2,2,6,6-tetramethylpiperidin-1-oxyl(4-OH-THEMPO)) und/oder Nitrosoverbindungen wie z. B. Nitrophenole (sowie alle anderen in der WO 00/64947 genannten Polymerisationsinhibitoren).

Die erfindungsgemäße Verfahrensweise ermöglicht es, den Inhibitorgehalt der flüssigen Monomerenphase bei sicherer Lagerung ebenso zu verringern wie den Sauerstoffgehalt in der über der flüssigen Phase im Lagerbehälter befindlichen Gasphase. Selbstredend hängt die erforderliche Menge des verwendeten Inhibitors auch von der Art des Inhibitors, von der Lagertemperatur, von der Art des Monomeren und von der spezifischen Reinheit der zu lagernden flüssigen Monomerenphase ab. Bezogen auf die in der erfindungsgemäß zu lagernden flüssigen Monomerenphase enthaltene Gewichtsmenge an Monomerem beträgt der Inhibitorgehalt in der Regel ≤ 220 Gew.-ppm.

Entnimmt man einem Lagerbehälter nach dem erfindungsgemäßen Verfahren gelagerte flüssige Monomerenphase, so wird diese zu entnehmende bzw. entnommene flüssige Monomerenphase vorab ihrer Weiterverwendung für eine chemische Reaktion oder zu Zwecken ihres Transports (eines Transports mit erhöhter Sicherheit) erfindungsgemäß vorteilhaft ebenfalls wenigstens einer Trennoperation zur Abtrennung von in der zu entnehmenden bzw. entnommenen flüssigen Monomerenphase gelöst (molekular und/oder kolloidal; visuell nicht sichtbar) enthaltenem Polymerisat des Monomeren unterworfen. D. h., vorliegende Anmeldung umfasst zusätzlich ein Verfahren, bei dem sich an das Verfahren der erfindungsgemäßen Lagerung ein Verfahren zur Entnahme von flüssiger Monomerenphase aus dem Lagerbehälter anschließt und die zu entnehmende flüssige Monomerenphase bei ihrer Entnahme und/oder nach ihrer Entnahme vorab ihrer Weiterverwendung für eine chemische Reaktion oder zu Zwecken ihres Transports auf dem Seeweg per Schiff, oder per Lastkraftwagen oder einem Schienenfahrzeug (d. h., auf der Straße oder auf der Schiene) (z. B. in einem Tank eines Tankfahrzeugs, z. B. eines Tankschiffs oder eines Tankwagens (als Tankmaterial wird häufig Edelstahl verwendet); das Fassungsvermögen des Tanks des letzteren (das von einem fluiden Medium einnehmbare Innenvolumen) beträgt in der Regel wenigstens 5 m³, häufig wenigstens 10 m³ und vielfach 15 bis 40 m³ bzw. 20 bis 30 m³; teilweise ist ihr Innenraum in wenigstens zwei, in der Regel wenigstens 3 oder wenigstens 4 gegeneinander völlig abgetrennte Kammern aufgeteilt, die voneinander unabhängig mit flüssiger Monomerenphase befüllt werden; das Füllvolumen mit flüssiger Monomerenphase beträgt typisch ca. 80 bis 90 Vol.-% des von einer fluiden Phase jeweils einnehmbaren Innenvolumens; das Fassungsvermögen des Tanks eines Tankschiffs geht über dasjenige des Tanks eines Tankwagens normalerweise weit hinaus) wenigstens einer Trennoperation zur Abtrennung wenigstens einer Teilmenge (vorzugsweise wenigstens 25 Gew.-%, besser wenigstens 50 Gew.-%, noch besser wenigstens 75 Gew.-% und am besten die Gesamtmenge) von darin gelöst enthaltenem Polymerisat (Oligomerisat) (insbesondere der in dieser Schrift genannten relativen Molekulargewichte) des Monomeren unterworfen wird. Die unerwünschte Ausbildung von derartigem insbesondere radikalischem Polymerisat kann auch bei Anwendung einer erfindungsgemäßen Lagerung nicht vollständig unterdrückt werden (in der Regel erfolgt die Lagerung bei Temperaturen ≤ 50 °C, vorzugsweise ≤ 40 bzw. ≤ 30 °C, jedoch oberhalb des Festpunktes der flüssigen Monomerenphase; typische Lagertemperaturen betragen 17 bis 25 °C). Detaillierte Untersuchungen haben zum Ergebnis geführt, dass sich solchermaßen in erfindungsgemäß gelagerter flüssiger Monomerenphase gelöst enthaltenes Polymerisat insbesondere bei Verwendung von erfindungsgemäß gelagerter flüssiger Monomerenphase für radikalische Polymerisationen sowohl auf den Verlauf der radikalischen Polymerisation als auch auf die Qualität des dabei resultierenden Polymerisats nachteilig auswirkt, selbst wenn die Gehaltsmengen nur geringfügig sind. Vorliegende Erfindung umfasst daher auch ein Verfahren der z. B. radikalischen Polymerisation, das dadurch gekennzeichnet ist, dass wie vorstehend beschrieben in einem Lagerbehälter gelagerte bzw. aus dem Lagerbehälter entnommene bzw. transportierte flüssige Monomerenphase oder deren Gemisch mit von der aus dem Lagerbehälter entnommenen flüssigen Monomerenphase verschiedenen wenigstens einfach ungesättigten (vorzugsweise ethylenisch) Verbindungen einpolymerisiert (z. B. radikalisch initiiert) wird. Als Trennoperationen kommen dabei alle diejenigen in Betracht, die bereits für das erfindungsgemäße Verfahren der Lagerung einer flüssigen Monomerenphase ausgeführt und empfohlen wurden. Alle dort gemachten Aussagen gelten hier in entsprechender Weise.

Selbstverständlich kann die Filtervorrichtung auch unmittelbar in die Entnahmeleitung bzw. an der Entnahmestelle in die Behälterwand eingebaut sein.

D. h., erfindungsgemäß bevorzugt wird auch dem Lagerbehälter zu entnehmende bzw. entnommene flüssige Monomerenphase einer mechanischen Trennoperation unterworfen, die zur Abtrennung von feinstteiligen Feststoffen von Flüssigkeiten geeignet ist. Diesbezüglich besonders geeignet sind auch hier filtrierende und/oder zentrifugierende Trennoperationen. Insbesondere kommen alle für das erfindungsgemäße Lagerungsverfahren empfohlenen Filtrationsmethoden in Betracht, weshalb die bezüglich des erfindungsgemäßen Lagerungsverfahrens in dieser Schrift individualisiert gemachten Aussagen zur Filtration in identischer Weise für eine Entnahmefiltration gelten und zutreffend sind. Selbstverständlich kann die Filtriervorrichtung auch unmittelbar in die Entnahmeleitung bzw. an der Entnahmestelle in die Behälterwand eingebaut sein.

Besonders bevorzugt werden dabei Filtermaterialien (Filtermedien, Filtertypen) eingesetzt, deren Abscheidegrad für Partikel mit einem Teilchendurchmesser von ≥ 10 µm wenigstens 90 %, vorzugsweise wenigstens 95 % beträgt.

Ganz besonders bevorzugt werden dabei Filtermaterialien (Filtermedien, Filtertypen) eingesetzt, deren Abscheidegrad für Partikel mit einem Teilchendurchmesser von ≥ 5 µm wenigstens 90 %, vorzugsweise wenigstens 95 % beträgt.

Selbstverständlich sind aber auch bereits Filtermaterialien (Filtermedien bzw. Filtertypen) wirksam, deren Abscheidegrad für Partikel mit einem Teilchendurchmesser ≥ 30 µm (bzw. ≥ 20 µm, bzw. ≥ 10 µm, bzw. ≥ 5 µm, bzw. ≥ 1 µm) wenigstens 70 %, vorzugsweise wenigstens 80 %, besonders bevorzugt wenigstens 90 % bzw. wenigstens 95 % und mehr beträgt.

Insbesondere können an dieser Stelle die ACCUGAF Filterbeutel der Firma Eaton der Modellart AGF 51, AGF 53 und AGF 55 eingesetzt werden. Selbstverständlich kommen an dieser Stelle aber auch die Kerzenfilter der Firma FUHR GmbH in Betracht. Beispielhaft erwähnt seien die acuraPrograd Kerzenfilter der Firma FUHR mit Abscheideraten von wenigstens 99,9 % für Partikeldurchmesser ≥ 5 µm.

Zweckmäßigerweise wird man bei der Entnahme von erfindungsgemäß gelagerter flüssiger Monomerenphase aus dem Lagertank so vorgehen, dass man die zu entnehmende flüssige Monomerenphase aus dem Lagertank zunächst als solche in einen Prüftank hinein entnimmt (selbstredend könnte die empfohlene Filtration auch bereits bei der Entnahme erfolgen (z. B. durch ein in der Entnahmeleitung bzw. an der Entnahmestelle im Lagerbehälter befindliches Filter)).

Aus dem Prüftank wird die dem Lagertank entnommene flüssige Monomerenphase dann ein- oder mehrfach aufeinanderfolgend herausgepumpt und z. B. über ein wie vorstehend beschriebenes Filtersystem in den Prüftank jeweils wieder rückgeführt. Nimmt die Abscheidemenge im Filtersystem (z. B. acuraPrograd Kerzenfilter mit Abscheideraten von 99,9 % für Partikeldurchmesser ≥ 5 µm in einem Mehrfach-Gehäuse) nicht mehr oder nicht mehr nennenswert zu, wird der Trennvorgang abgebrochen und die im Prüftank befindliche, dem Lagertank zuvor entnommene Monomerenphase ihrer Weiterverwendung (z. B. ihrem Transport mit erhöhter Sicherheit zum Verbraucher, oder einer radikalischen Polymerisation oder einer anderen chemischen Reaktion) zugeführt. Vorzugsweise erfolgt auch vorbeschriebene Trennoperation bei möglichst geringer Temperatur (in der Regel ≤ 95 °C, besser ≤ 80 °C, bevorzugt ≤ 60 °C, mit Vorteil ≤ 40 °C, besser bevorzugt ≤ 30 °C bzw. ≤ 25 °C und in günstigen Fällen bis zu 0 °C).

Die vorliegende Anmeldung umfasst daher auch ein Verfahren des Transports einer flüssigen Monomerenphase, die gemäß eines erfindungsgemäßen Verfahrens in einem Lagerbehälter gelagert und gemäß einer erfindungsgemäßen Entnahme aus dem Lagerbehälter entnommen worden ist.

Im übrigen kann die Lagerung der flüssigen Monomerenphase so erfolgen, wie es die WO 2005/049543, die US 6,910,511, Research Disclosure Database Number 513002, Published in January 2007 und insbesondere Research Disclosure Database Number 513001, Published in January 2007, beschreiben.

Erfolgt die Kondensation zur Erzeugung der erfindungsgemäß zu lagernden flüssigen Monomerenphase in einer Rektifikationskolonne oberhalb der Zufuhrstelle (Zulaufstelle) des (in der Rektifikationskolonne) aufzutrennenden Gemischs in die Rektifikationskolonne aus den in der Rektifikationskolonne aufsteigenden Dämpfen heraus, und wird die so kondensativ erzeugte und erfindungsgemäß zu lagernde flüssige Monomerenphase dann oberhalb des vorgenannten Zulaufs aus der Rektifikationskolonne entnommen, kann die Kondensation innerhalb der Rektifikationskolonne z. B. durch direkte Kühlung und/oder indirekte Kühlung bewirkt werden. Eine indirekte Kühlung kann z. B. am Kolonnenkopf dadurch bewirkt werden, dass man den am Kolonnenkopf ankommenden aufgestiegenen Dampf durch einen indirekten Wärmeaustauscher führt und die unter den Bedingungen des indirekten Wärmeaustauschs kondensierbaren Bestandteile in die flüssige Phase überführt. Ein Teil der so erzeugten flüssigen Phase wird dann der erfindungsgemäßen Lagerung zugeführt und der andere Teil als Rücklaufflüssigkeit auf den Kopf der Rektifikationskolonne rückgeführt. Diese Rücklaufflüssigkeit bedingt dann in der Rektifikationskolonne bereits eine direkte Kühlung des in der Rektifikationskolonne aufsteigenden Dampfes. Im indirekten Wärmeaustauscher nicht kondensierbare Bestandteile werden aus der Rektifikationskolonne heraus- und in der Regel ihrer Entsorgung zugeführt. Der Polymerisationsinhibitor wird unmittelbar ins Kondensat zugegeben. Erfolgt die Reinproduktentnahme unterhalb des Kolonnenkopfes, wird die am Kopf kondensierte Phase normalerweise weitgehend als Rücklaufflüssigkeit in die Rektifikationskolonne rückgeführt. Üblicherweise enthält die Rücklaufflüssigkeit Polymerisationsinhibitor zugesetzt. Das aus der Rektifikationskolonne entnommene Reinprodukt ist so unmittelbar ebenfalls polymerisationsinhibiert. Selbstredend kann die Kondensation am Kolonnenkopf auch ausschließlich durch direkte Kühlung vorgenommen werden. Zu diesem Zweck wird einmal erzeugtes Kopfkondensat mit Inhibitor versetzt, abgekühlt und wenigstens teilweise in den Kopfraum der Rektifikationskolonne zum Zweck der Direktkühlung versprüht. Erfolgt die Reinproduktentnahme unterhalb des Kolonnenkopfes (aber oberhalb der Zufuhrstelle des aufzutrennenden Gemischs in die Rektifikationskolonne) wird normalerweise die abgekühlte Kondensatgesamtmenge in den Kopfraum rückversprüht. Der trennwirksame Einbauten enthaltende Teil der Rektifikationskolonne und der Kondensationsraum am Kolonnenkopf sind üblicherweise durch einen Kaminboden voneinander getrennt. Die Rücklaufflüssigkeit wird dem trennwirksamen Teil zugeführt. Der unterhalb der Zufuhrstelle (Zulaufstelle) in die Rektifikationskolonne befindliche Teil der Rektifikationskolonne wird üblicherweise als Abtriebsteil und der oberhalb der Zufuhrstelle befindliche Teil der Rektifikationskolonne wird üblicherweise als Verstärkerteil der Rektifikationskolonne bezeichnet.

Erfolgt die Zufuhr des in der Rektifikationskolonne aufzutrennenden Stoffgemischs in die Rektifikationskolonne in flüssiger Form, bildet die Auftrennung eine Rektifikation, erfolgt die Zufuhr dampfförmig (bzw. gasförmig), bildet die Auftrennung eine fraktionierende Kondensation. Als trennwirksame Einbauten kann die Rektifikationskolonne z. B. Böden, Packungen und/oder Füllkörper enthalten. Beispielhafte Ausführungsformen einer fraktionierenden Kondensation von Acrylsäure aus dem Produktgasgemisch einer heterogen katalysierten partiellen Gasphasenoxidation von Propylen und/oder Propan offenbaren z. B. die Schriften DE-A 199 24 533, DE-A 199 24 532, WO 01/77056, DE-A 101 56 016, DE-A 102 43 625, DE-A 102 23 058, DE-A 102 35 847, WO 2004/035514, WO 00/53560 und DE-A 103 32 758.

Wird die erfindungsgemäß zu lagernde Monomerenphase durch Aufschmelzen einer kristallinen Phase erzeugt, kann diese das Ergebnis einer einstufigen oder einer mehrstufigen kristallisativen Reinigung eines das relevante Monomere enthaltenden flüssigen Gemisches sein (vgl. z. B. EP-A 616 998). Durch Einwirkung von Kälte auf das flüssige Gemisch kristallisiert das Monomere aus dem flüssigen Gemisch als kristalline Phase aus.

Häufig ist die aufzuschmelzende kristalline Phase das Ergebnis einer einstufigen Kristallisation.

Selbstverständlich kann die aufzuschmelzende Phase auch das Ergebnis einer fraktionierten Kristallisation sein.

Beispielsweise kann die Kälteeinwirkung auf das das Monomere enthaltende flüssige Gemisch als Schichtkristallisation ausgeführt sein (vgl. DE-OS 26 06 364, EP-A 616 998, EP-A 648 520 und EP-A 776 875). Dabei wird das Kristallisat in Form zusammenhängender, fest anhaftender Schichten an gekühlten Oberflächen ausgefroren. Die Trennung des abgeschiedenen Kristallisats von der verbliebenen Restschmelze (der Mutterlauge) erfolgt durch einfaches Abfließen der Restschmelze. Prinzipiell unterscheidet man zwischen "statischen" und "dynamischen" Schichtkristallisationsverfahren. Kennzeichnend für die dynamische Schichtkristallisation aus flüssigen Gemischen ist eine erzwungene Konvektion des flüssigen Gemischs. Diese kann z. B. durch Umpumpen des flüssigen Gemischs durch gekühlte volldruchströmte Rohre, durch Aufgabe des flüssigen Gemischs als Rieselfilm auf gekühlte Wandungen (z. B. gemäß EP-A 616 998, z. B. in gekühlten Fallrohen) oder durch Einleiten von Inertgas in das flüssige Gemisch oder durch Pulsieren erfolgen.

Bei den statischen Verfahren ruht das flüssige Gemisch (z. B. in Rohrbündel oder Plattenwärmeaustauschern) und scheidet sich schichtförmig durch langsame Temperatursenkung auf der Sekundärseite des Wärmeaustauschers ab. Danach wird die Restschmelze (Mutterlauge) abgelassen, durch langsame Temperaturerhöhung stärker verunreinigte Fraktionen aus der Kristallschicht abgeschwitzt und nachfolgend das Reinprodukt abgeschmolzen (vgl. WO 01/77056).

Generell enthält das das Monomere enthaltende flüssige Gemisch (normalerweise eine Lösung), aus dem das Monomere kristallisativ abgetrennt wird, Polymerisationsinhibitor zugesetzt. Bei der kristallisativen Abscheidung des Monomeren kristallisiert dieses normalerweise im wesentlichen frei vom Polymerisationsinhibitor aus. Das Aufschmelzen der abgeschiedenen Monomerenkristallschicht erfolgt daher häufig nicht durch Erwärmen der Sekundärseite, sondern durch in Kontakt bringen der Monomerenkristallschicht mit einer Polymerisationsinhibitor zugesetzt enthaltenden erwärmten Schmelze von zuvor aufgeschmolzener abgeschiedener Monomerenkristallschicht.

Alternativ kann die Ausbildung des Monomerenkristallisats aus dem das Monomere enthaltenden flüssigen Gemisch auch als Suspensionskristallisation (z. B. gemäß der Lehre der WO 01/77056, der WO 02/055469 sowie der WO 03/078378) erfolgen.

Dabei wird in der Regel durch Kühlung des z. B. Acrylsäure als Monomeres enthaltenden flüssigen Gemischs (der flüssigen Lösung) eine Acrylsäurekristalle suspendiert enhaltende Kristallsuspension erzeugt, wobei die Acrylsäurekristalle einen geringeren und die verbliebene Restschmelze (Mutterlauge) einen höheren Verunreinigungsgehalt aufweist als das flüssige Ausgangsgemisch. Dabei können die Acrylsäurekristalle unmittelbar in Suspension befindlich wachsen und/oder sich als Schicht auf einer gekühlten Wand abscheiden, von der sie stetig abgekratzt und in der Restschmelze resuspendiert werden.

Alle in der WO 01/77056, der WO 02/055469 sowie in der WO 03/078378 ausgeführten Suspensionskristaller und Suspensionskristallisationsverfahren können dabei angewendet werden. Eine so erzeugte Acrylsäurekristallisatsuspension kann z. B. einen Feststoffgehalt von 10 bis 50, häufig 20 bis 40 oder 30 Gew.-% aufweisen.

Zur Trennung von Suspensionskristallisat und verbliebener Mutterlauge kommen z. B. alle in den vorgenannten WO-Veröffentlichungen genannten Trennverfahren in Betracht (z. B. mechanische Trennverfahren wie Zentrifugieren). Bevorzugt erfolgt die Trennung in einer Waschkolonne. Mit Vorteil handelt es sich dabei um eine Waschkolonne mit erzwungenem Transport der abgeschiedenen z. B. Acrylsäurekristalle. Als Waschflüssigkeit wird mit Vorteil die Schmelze von in der Waschkolonne vorab gereinigten (abgetrennten) z. B. Acrylsäurekristallen verwendet. Die Wäsche erfolgt normalerweise im Gegenstrom.

Alles Vorgenannte gilt vor allem dann, wenn die Waschkolonne eine Waschkolonne mit erzwungenem Transport der z. B. Acrylsäurekristalle ist, und dies vor allem dann, wenn es eine hydraulische oder eine mechanische Waschkolonne gemäß der WO 01/77056 ist und sie wie dort ausgeführt betrieben wird.

Eine erfindungsgemäß zu lagernde flüssige Acrylsäurephase kann damit z. B. wie folgt erzeugt werden. Durch heterogen katalysierte einstufige oder zweistufige Partialoxidation eines C₃-Vorläufers der Acrylsäure (z. B. Propylen, Propan oder Acrolein) wird ein Acrylsäure enthaltendes Produktgasgemisch erzeugt. Dieses wird in eine Trennkolonne mit trennwirksamen Einbauten geführt und in selbiger aufsteigend fraktionierend kondensiert. Oberhalb der Zufuhrstelle aber unterhalb des Kolonnenkopfes wird aus der Trennkolonne eine flüssige rohe Acrylsäure entnommen, die ≥ 95 Gew.-% an Acrylsäure aufweist und über den Rücklauf in der Trennkolonne Polymerisationsinhibitor zugesetzt enthält. Durch Suspensionskristallisation wird aus der rohen Acrylsäure Reinacrylsäuresuspensionskristallisat erzeugt. Dieses wird in einer Waschkolonne (vorzugsweise in einer hydraulischen Waschkolonne) unter Verwendung von Polymerisationsinhibitor zugesetzt enthaltender Reinkristallisatschmelze als Waschflüssigkeit von verbliebener Mutterlauge abgetrennt. Durch in Kontakt bringen von abgetrenntem Reinkristallisat mit der Polymerisationsinhibitor zugesetzt enthaltenden Schmelze von zuvor abgetrenntem Reinkristallisat wird das abgetrennte Reinkristallisat aufgeschmolzen und so eine erfindungsgemäß zu lagernde flüssige Acrylsäurephase erzeugt (Reinheit in der Regel ≥ 99,5 Gew.-%).

Selbstverständlich können die in dieser Schrift ausgeführten Verfahren der Lagerung und der Entnahme auch auf flüssige Monomerenphasen angewendet werden, die auf andere Art und Weise erzeugt wurden und deren Gehalt an dem Monomeren < 95 Gew.-% beträgt. Das erfindungsgemäße Verfahren kann auch so durchgeführt werden, dass man die zu lagernde flüssige Monomerenphase (gegebenenfalls zunächst als solche) in den Lagerbehälter führt und dann dem Lagertank im Verlauf der Lagerung immer wieder (am besten kontinuierlich) flüssige Monomerenphase entnimmt und diese anschließend wieder in den Lagertank rückführt und auf diesem Weg der Entnahme und Rückführung wenigstens einer Trennoperation zur Abtrennung wenigstens einer Teilmenge von in der flüssigen Monomerenphase gelöst enthaltenem Polymerisat des Monomeren unterwirft. In diesem Fall führt der erfindungsgemäße Weg der flüssigen Monomerenphase von ihrer Erzeugung in den Lagerbehälter zunächst in einfacher Weise über eine Schlaufe der zwischenzeitlichen nochmaligen Herausführung aus dem Lagerbehälter. Selbstverständlich können alle in dieser Schrift vorgestellten erfindungsgemäßen Varianten (oder Teilmenge dieser Varianten) auch kombiniert angewendet werden.

### Beispiel und Vergleichsbeispiel

### a) Vergleichsbeispiel

Durch fraktionierende Kondensation des Produktgasgemischs einer heterogen katalysierten partiellen Propylenoxidation wurde wie in Beispiel 1 der WO 2004/035514 beschrieben eine Acrylsäure erzeugt, die 96,9 Gew.-% Acrylsäure enthielt und mit 0,018 Gew.-% MEHQ sowie 0,012 Gew.-% Phenothiazin und 0,0004 Gew.-% molekularem Sauerstoff polymerisationsinhibiert war. Sie wurde vom zweiten Fangboden der Kondensationskolonne oberhalb der Zufuhr des Produktgasgemischs in die Kondensationskolonne mit einer Temperatur von 100,6 °C und visuell frei von Feststoffen aus der Kondensationskolonne entnommen. 0,5 ml dieser Acrylsäure wurden auf 25 °C abgekühlt und unter Luftatmosphäre in eine 1,8 ml Glasampulle überführt. Anschließend wurde die Ampulle bei 120 °C im Umlufttrockenschrank drehend gelagert, um vollständige Durchmischung zu gewährleisten. Dann wurde die Zeit t bis zu vollständigen Polymerisation der Probe erfasst. t betrug 15 h 17 Minuten.

### b) Beispiel

Es wurde wie im Vergleichsbeispiel verfahren. Nach ihrer Entnahme aus der Trennkolonne wurde die Acrylsäure auf 30 °C abgekühlt und durch in einem Mehrfach-Gehäuse befindliche ACCUGAF AGF-51 Filterbeutel geführt. Nach beendeter Filtration wiesen die Innenwände der Filterbeutel einen klebrigen Belag aus Polyacrylsäure auf. 0,5 ml der filtrierten Acrylsäure wurden mit 25 °C und unter Luftatmosphäre in eine 1,8 ml Glasampulle überführt. Anschließend wurde die Ampulle bei 120 °C im Umlufttrockenschrank drehend gelagert, um vollständige Durchmischung zu gewährleisten. Dann wurde die Zeit t bis zur vollständigen Polymerisation der Probe erfasst. t betrug 20 h 26 Minuten.

Wie die beiden Versuche ausweisen, mindert die Entfernung von gelöst enthaltenem Polymerisat (gelöst enthaltener Polyacrylsäure) die Neigung der Acrylsäure zu unerwünschter radikalischer Polymerisation.

## Patentansprüche

1. Verfahren zur Lagerung einer unter den Bedingungen der Lagerung flüssigen Monomerenphase, deren Gehalt an dem Monomeren ≥ 95 Gew.-% beträgt, in einem Lagerbehälter, wobei das Monomere ein Monomeres aus der Gruppe bestehend aus Acrolein, Methacrolein, Acrylsäure, Methacrylsäure, Ester aus Acrylsäure und einem 1 bis 12 C-Atome aufweisenden Alkohol sowie Ester aus Methacrylsäure und einem 1 bis 12 C-Atome aufweisenden Alkohol ist und die flüssige Monomerenphase durch Kondensation aus einer gasförmigen Phase heraus oder durch Aufschmelzen einer kristallinen Phase erzeugt wurde, **dadurch gekennzeichnet, dass** die flüssige Monomerenphase auf dem Weg von ihrer Erzeugung in den Lagerbehälter wenigstens einer Trennoperation zur Abtrennung wenigstens einer Teilmenge von in der flüssigen Monomerenphase gelöst enthaltenem Polymerisat des Monomeren unterworfen wird und der Lagerbehälter eine Vorrichtung aufweist, mit Hilfe derer eine Teilmenge der gelagerten flüssigen Monomerenphase entnommen, über einen Wärmeaustauscher geführt und anschließend in den Lagertank rückgeführt werden kann.

2. Verfahren nach Anspruch 1, **dadurch gekennzeichnet, dass** das von einer fluiden Phase einnehmbare Innenvolumen des Lagerbehälters 100 m³ bis 10000 m³ beträgt.

3. Verfahren nach einem der Ansprüche 1 bis 2, **dadurch gekennzeichnet, dass** die flüssige Monomerenphase im Lagerbehälter unter einer molekularen Sauerstoff enthaltenden Atmosphäre gelagert wird.

4. Verfahren nach einem der Ansprüche 1 bis 3, **dadurch gekennzeichnet, dass** die flüssige Monomerenphase p-Methoxyphenol und/oder Phenothiazin gelöst enthält.

5. Verfahren nach einem der Ansprüche 1 bis 4, **dadurch gekennzeichnet, dass** die wenigstens eine Trennoperation das Filtrieren und/oder das Zentrifugieren ist.

6. Verfahren nach einem der Ansprüche 1 bis 5, **dadurch gekennzeichnet, dass** die wenigstens eine Trennoperation eine Filtration ist, bei der das verwendete Filtermedium aus Polypropylen gefertigt ist.

7. Verfahren nach einem der Ansprüche 1 bis 5, **dadurch gekennzeichnet, dass** die wenigstens eine Trennoperation eine Filtration ist, bei der das Filtermedium aus Edelstahl gefertigt ist.

8. Verfahren nach einem der Ansprüche 1 bis 7, **dadurch gekennzeichnet, dass** die wenigstens eine Trennoperation eine Filtration ist, bei der der Abscheidegrad des eingesetzten Filtriermediums für Partikel mit einem Teilchendurchmesser ≥ 30 µm wenigstens 90% beträgt.

9. Verfahren nach einem der Ansprüche 1 bis 8, **dadurch gekennzeichnet, dass** die wenigstens eine Trennoperation eine Filtration ist, bei der der Abscheidegrad des eingesetzten Filtriermediums für Partikel mit einem Teilchendurchmesser ≥ 10 µm wenigstens 90% beträgt.

10. Verfahren nach einem der Ansprüche 1 bis 9, **dadurch gekennzeichnet, dass** die wenigstens eine Trennoperation bei einer Temperatur von ≤ 95°C durchgeführt wird.

11. Verfahren nach einem der Ansprüche 1 bis 10, **dadurch gekennzeichnet, dass** sich ein Verfahren zur Entnahme von flüssiger Monomerenphase aus dem Lagerbehälter anschliesst und die zu entnehmende flüssige Monomerenphase bei ihrer Entnahme und/oder nach ihrer Entnahme vorab ihrer Weiterverwendung für eine chemische Reaktion oder zu Zwecken ihres Transports wenigstens einer Trennoperation zur Abtrennung von darin gelöst enthaltenem Polymerisat des Monomeren unterworfen wird.

12. Verfahren nach Anspruch 11, **dadurch gekennzeichnet, dass** die wenigstens eine Trennoperation eine Filtration ist.

13. Verfahren nach Anspruch 12, **dadurch gekennzeichnet, dass** der Abscheidegrad des eingesetzten Filtriermediums für Partikel mit einem Teilchendurchmesser ≥ 10 µm wenigstens 90% beträgt.

14. Verfahren nach einem der Ansprüche 1 bis 13, **dadurch gekennzeichnet, dass** die flüssige Monomerenphase eine Reinacrylsäure ist, deren Acrylsäuregehalt ≥ 99 Gew.-% beträgt.

15. Verfahren der radikalischen Polymerisation, **dadurch gekennzeichnet, dass** gemäß eines Verfahrens nach einem der Ansprüche 11 bis 12 aus dem Lagerbehälter entnommene flüssige Monomerenphase oder deren Gemisch mit von der aus dem Lagerbehälter entnommenen flüssigen Monomerenphase verschiedenen wenigstens einfach ungesättigten Verbindungen einpolymerisiert wird.

16. Verfahren des Transports einer flüssigen Monomerenphase, **dadurch gekennzeichnet, dass** die flüssige Monomerenphase eine gemäß eines Verfahrens nach einem der Ansprüche 11 bis 13 aus dem Lagerbehälter entnommene flüssige Monomerenphase ist.

## Claims

1. A process for storing a monomer phase which is liquid under the conditions of storage and whose content of the monomer is ≥ 95% by weight in a storage vessel, the monomer being a monomer from the group consisting of acrolein, methacrolein, acrylic acid, methacrylic acid, esters of acrylic acid and an alcohol having from 1 to 12 carbon atoms and esters of methacrylic acid and an alcohol having from 1 to 12 carbon atoms, and the liquid monomer phase having been obtained by condensation out of a gaseous phase or by melting of a crystalline phase, wherein the liquid monomer phase, on the route from its generation into the storage vessel, in subjected to at least one separating operation to remove at least a portion of polymer of the monomer present dissolved in the liquid monomer phase and the storage vessel has a device with the aid of which a portion of the stored liquid monomer phase can be withdrawn, conducted through a heat exchanger and then recycled into the storage tank.

2. The process according to claim 1, wherein the internal volume of the storage vessel occupiable by a fluid phase is from 100 m³ to 10 000 m³.

3. The process according to either of claims 1 and 2, wherein the liquid monomer phase in the storage vessel is stored under an atmosphere comprising molecular oxygen.

4. The process according to any of claims 1 to 3, wherein the liquid monomer phase comprises dissolved p-methoxyphenol and/or phenothiazine.

5. The process according to any of claims 1 to 4, wherein the at least one separating operation is filtration and/or centrifugation.

6. The process according to any of claims 1 to 5, wherein the at least one separating operation is a filtration in which the filter medium used has been manufactured from polypropylene.

7. The process according to any of claims 1 to 5, wherein the at least one separating operation is a filtration in which the filter medium has been manufactured from stainless steel.

8. The process according to any of claims 1 to 7, wherein the at least one separating operation is a filtration in which the degree of retention efficiency of the filter medium used for particles having a particle diameter of ≥ 30 µm is at least 90%.

9. The process according to any of claims 1 to 8, wherein the at least one separating operation is a filtration in which the degree of retention efficiency of the filter medium used for particles having a particle diameter of ≥ 10 µm is at least 90%.

10. The process according to any of claims 1 to 9, wherein the at least one separating operation is performed at a temperature of ≤ 95°C.

11. The process according to any of claims 1 to 10, which is followed by a process for withdrawing liquid monomer phase from the storage vessel and the liquid monomer phase to be withdrawn, when it is withdrawn and/or after it has been withdrawn and before its further use for a chemical reaction or for the purpose of its transport, is subjected to at least one separating operation to remove polymer of the monomer which is present dissolved therein.

12. The process according to claim 11, wherein the at least one separating operation is a filtration.

13. The process according to claim 12, wherein the degree of retention efficiency of the filter medium used for particles having a particle diameter of ≥10 µm is at least 90%.

14. The process according to any of claims 1 to 13, wherein the liquid monomer phase is glacial acrylic acid whose acrylic acid content is ≥99% by weight.

15. A process for free-radical polymerization, which comprises polymerizing liquid monomer phase withdrawn from the storage vessel by a process according to either of claims 11 and 12 or a mixture thereof with at least monounsaturated compounds other than the liquid monomer phase withdrawn from the storage vessel.

16. A process for transporting a liquid monomer phase, wherein the liquid monomer phase is a liquid monomer phase withdrawn from the storage vessel by a process according to any of claims 11 to 13.

## Revendications

1. Procédé de stockage d'une phase monomère liquide dans les conditions du stockage, dont la teneur en monomère est ≥ 95 % en poids, dans un contenant de stockage, le monomère étant un monomère du groupe constitué par l'acroléine, la méthacroléine, l'acide acrylique, l'acide méthacrylique, les esters de l'acide acrylique et d'un alcool comprenant 1 à 12 atomes C, ainsi que les esters de l'acide méthacrylique et d'un alcool comprenant 1 à 12 atomes C, et la phase monomère liquide ayant été formée par condensation à partir d'une phase gazeuse ou par fusion d'une phase cristalline, **caractérisé en ce que** la phase monomère liquide est soumise sur son trajet entre sa formation et le contenant de stockage à au moins une opération de séparation pour la séparation d'au moins une partie du polymère du monomère contenu sous forme dissoute dans la phase monomère liquide, et le contenant de stockage comprend un dispositif à l'aide duquel une partie de la phase monomère liquide stockée peut être soutirée, conduite sur un échangeur de chaleur, puis recyclée dans la cuve de stockage.

2. Procédé selon la revendication 1, **caractérisé en ce que** le volume intérieur du contenant de stockage occupable par une phase fluide est de 100 m³ à 10 000 m³.

3. Procédé selon l'une quelconque des revendications 1 à 2, **caractérisé en ce que** la phase monomère liquide est stockée dans le contenant de stockage sous une atmosphère contenant de l'oxygène moléculaire.

4. Procédé selon l'une quelconque des revendications 1 à 3, **caractérisé en ce que** la phase monomère liquide contient du p-méthoxyphénol et/ou de la phénothiazine.

5. Procédé selon l'une quelconque des revendications 1 à 4, **caractérisé en ce que** ladite au moins une opération de séparation est une filtration et/ou une centrifugation.

6. Procédé selon l'une quelconque des revendications 1 à 5, **caractérisé en ce que** ladite au moins une opération de séparation est une filtration lors de laquelle le milieu de filtration utilisé est en polypropylène.

7. Procédé selon l'une quelconque des revendications 1 à 5, **caractérisé en ce que** ladite au moins une opération de séparation est une filtration lors de laquelle le milieu de filtration est en acier inoxydable.

8. Procédé selon l'une quelconque des revendications 1 à 7, **caractérisé en ce que** ladite au moins une opération de séparation est une filtration lors de laquelle le degré de séparation du milieu de filtration utilisé pour les particules ayant un diamètre de particule ≥ 30 µm est d'au moins 90 %.

9. Procédé selon l'une quelconque des revendications 1 à 8, **caractérisé en ce que** ladite au moins une opération de séparation est une filtration lors de laquelle le degré de séparation du milieu de filtration utilisé pour les particules ayant un diamètre de particule ≥ 10 µm est d'au moins 90 %.

10. Procédé selon l'une quelconque des revendications 1 à 9, **caractérisé en ce que** ladite au moins une opération de séparation est réalisée à une température ≤ 95 °C.

11. Procédé selon l'une quelconque des revendications 1 à 10, **caractérisé en ce qu'**un procédé de soutirage de la phase monomère liquide du contenant de stockage s'ensuit et la phase monomère liquide à soutirer est soumise lors de son soutirage et/ou après son soutirage avant son utilisation ultérieure pour une réaction chimique ou pour son transport à au moins une opération de séparation pour la séparation du polymère du monomère contenu sous forme dissoute dans celle-ci.

12. Procédé selon la revendication 11, **caractérisé en ce que** ladite au moins une opération de séparation est une filtration.

13. Procédé selon la revendication 12, **caractérisé en ce que** le degré de séparation du milieu de filtration utilisé pour les particules ayant un diamètre de particule ≥ 10 µm est d'au moins 90 %.

14. Procédé selon l'une quelconque des revendications 1 à 13, **caractérisé en ce que** la phase monomère liquide est de l'acide acrylique pur dont la teneur en acide acrylique est ≥ 99 % en poids.

15. Procédé de polymérisation radicalaire, **caractérisé en ce qu'**une phase monomère liquide soutirée du contenant de stockage par un procédé selon l'une quelconque des revendications 11 à 12 ou son mélange avec des composés au moins monoinsaturés différents de la phase monomère liquide soutirée du contenant de stockage est polymérisé.

16. Procédé de transport d'une phase monomère liquide, **caractérisé en ce que** la phase monomère liquide est une phase monomère liquide soutirée du contenant de stockage par un procédé selon l'une quelconque des revendications 11 à 13.
